Europäisches Patentamt

**European Patent Office**

**Office européen des brevets**

(11) Numéro de publication: **0 097 580**
**A1**

(12) **DEMANDE DE BREVET EUROPEEN**

(21) Numéro de dépôt: **83401232.0**

(22) Date de dépôt: **15.06.83**

(51) Int. Cl.³: **A 61 K 37/54**
//(A61K37/54, 37/02, 31/71)

(30) Priorité: **16.06.82 FR 8210927**

(43) Date de publication de la demande:
**04.01.84 Bulletin 84/1**

(84) Etats contractants désignés:
**AT BE CH DE FR GB IT LI LU NL SE**

(71) Demandeur: **Lacombe, Jean-François Paul Louis**

**F-50680 Cerisy la Foret(FR)**

(72) Inventeur: **Lacombe, Jean-François Paul Louis**

**F-50680 Cerisy la Foret(FR)**

(74) Mandataire: **Lavoix, Jean et al,**
**c/o Cabinet Lavoix 2, Place D'Estienne D'Orves**
**F-75441 Paris Cedex 09(FR)**

(54) **Nouveau médicament vétérinaire destiné au traitement des mammites infectieuses des bovins et caprins.**

(57) Nouveau médicament à usage vétérinaire à base de deux antibiotiques associés à une enzyme et possédant une activité antimicrobienne.

Les antibiotiques sont la polymyxine et l'érythromycine ou leurs sels pharmaceutiquement acceptables ; l'enzyme est le lysozyme ou ses sels pharmaceutiquement acceptables .

La présente invention est particulièrement utile pour l'espèce bovine et l'espèce caprine dans le traitement des mammites aiguës, subaiguës, récidivantes, d'étiologie infectieuse.

EP 0 097 580 A1

Croydon Printing Company Ltd.

# Nouveau médicament vétérinaire destiné au traitement des mammites infectieuses des bovins et caprins

La présente invention est relative à un nouveau médicament à usage vétérinaire, caractérisé en ce qu'il contient deux antibiotiques associés à une enzyme, et destiné à traiter les affections inflammatoires des glandes mammaires bovines et caprines. Dans le domaine vétérinaire, différentes associations d'antibiotiques ont été décrites ; ainsi, le brevet français 2186228 décrit des associations de tylosine et de colistine ou d'érythromycine et de colistine, compositions ayant une large activité antimicrobienne pour les ruminants, les porcs et les volailles. Les affections des glandes mammaires bovines et caprines restent un des problèmes majeurs des producteurs de lait depuis le déclin de la brucellose. L'étiologie de ces troubles est d'origine généralement microbienne et de nombreux travaux ont été menés dans le but de diminuer le fléau dont le coût global pour l'agriculture est particulièrement important. Ces travaux ont porté soit sur la prémunition du cheptel laitier par la voie de la vaccination, soit sur la diminution du microbisme ambiant et en particulier par une désinfection systématique des trayons (ces pratiques n'ont pu diminuer le taux des tes qu'à peine de 20 p.100), soit sur l'imprégnation de parenchyme mammaire par des substances antibiotiques au moment de la période de tarissement. Cette dernière pratique certes est efficace sur les mammites latentes. Reste le traitement des mammites lors de la lactation qui, à l'heure actuelle, présente les inconvénients suivants :

- faible diffusion du ou des principes actifs, les canaux excréteurs de la mamelle malade étant souvent obstrués par des amas fibrineux,

- pollution du lait du quartier traité par des résidus d'antibiotiques pendant au moins 70 à 120 heures, la réglementation interdisant la commercialisation de ce lait pour la consommation,

z

- nécessité de multiplier les injections intramammaires du produit traitant pour obtenir un bon effet antibactérien.

La présente invention a pour objet de pallier l'ensemble de ces inconvénients.

Le médicament de l'invention est ainsi composé de
deux antibiotiques, la polymyxine et l'érythromycine, en association avec une enzyme, le lysozyme.

Dans cette composition, les antibiotiques base et
l'enzyme peuvent être remplacés par leurs sels avec des acides
organiques ou minéraux pharmaceutiquement acceptables.

La polymyxine avantageusement utilisée dans l'invention peut être la POLYMYXINE B ou la POLYMYXINE E (ou COLISTINE).

Le sulfate de polymyxine B, qui est inscrit à la
Pharmacopée européenne, peut être défini comme étant un mélange de sulfates de polypeptides élaboré par certaines souches
de BACILLUS POLYMYXA ou obtenu par tout autre moyen. Il se présente sous forme d'une poudre blanche ou blanc crème, sensiblement inodore, hygroscopique, très soluble dans l'eau, peu soluble dans l'alcool. Cet antibiotique est connu pour son activité bactéricide sur la plupart des bacilles gram (-), à l'exception des PROTEUS. Il agit particulièrement contre ESCHERICHIA
COLI, AEROBACTER AEROGENES ENTEROBACTER, SERRATIA et PSEUDOMONAS AERUGINOSA ainsi que sur KLEBSIELLA. Administré à la souris,
la DLSQ/24 h/kg se situe à 1107 mg pour la voie orale, à 24 mg
pour la voie intra-péritonéale, à 250-300 mg pour la voie sous-
cutanée, à 34 mg pour la voie intra-musculaire et à 6,8 mg pour
la voie endo-veineuse. Chez le rat, la DL50/24 h/kg se situe à
9 mg quand l'antibiotique est administré par voie intra-veineuse.
Quand la dose ne dépasse pas 2,5 mg/kg/24 h, la POLYMYXINE est
dénuée de tout phénomène néphrotoxique ; cet antibiotique est
également dénué de toute action sur l'intestin isolé et sur le
système nerveux du chien recevant 1 mg/kg/i.v. en une seule dose. Doué d'une action très rapide, il agit sur les bactéries
en phase active de multiplication ou en phase de repos par lyse

0097580

3

osmotique de la membrane cellulaire, en se fixant par ses groupements libres $NH_2$ sur les sites amioniques de la membrane. Il est très rapidement absorbé par voie intra-musculaire, sa demi-vie plasmatique étant de 4 heures environ. En raison de sa grande molécule, il ne franchit pas les barrières tissulaires et, en particulier, il ne pénètre ni dans le liquide céphalo-rachidien ni dans les cavités séreuses. Métabolisé à 50 %, il est éliminé à 80 % de la dose administrée en 24 heures par filtration glomérulaire dans les urines. Il est classiquement utilisé pour les infections intestinales et les salmonelloses digestives et pour la stérilisation pré- et post-opératoire du tube digestif. Il est également indiqué pour le traitement des infections viscérales, les septicémies et les méningites.

Le sulfate de polymyxine E ou sulfate de COLISTINE, qui peut être utilisé en lieu et place de la polymyxine B dans le médicament de l'invention est également inscrit à la Pharmacopée Française. Il est défini comme étant un mélange de sulfates de polypeptides élaboré par certaines souches de BACILLUS POLYMYXA, variété COLISTINUS, ou obtenu par tout autre moyen.

Le sulfate de polymyxine E possède les mêmes caractéristiques organoleptiques et de solubilité que le sulfate de polymyxine B ; son activité bactéricide et son métabolisme sont également identiques.

L'érythromycine est une substance élaborée par certaines souches de STREPTOMYCES ERYTHREUS. Elle est inscrite à la Pharmacopée européenne. Son thiocyanate se présente sous forme d'une poudre pratiquement blanche, soluble dans l'eau, l'alcool et le chloroforme. Ce sel possède les mêmes activités que la base et il est utilisé en médecine vétérinaire dans l'eau de boisson médicamenteuse. Son activité bactériostatique et bactéricide dépend de la concentration utilisée. Son activité augmente également en fonction du pH jusqu'à pH 8,5 environ. Son activité antimicrobienne est proche de celle de la pénicilline. Elle agit sur presque toutes les bactéries gram (+) et en particulier sur les staphylocoques, les streptocoques certains entérocoques, et sur

4

Corynebactèrium pyogenes. Sur une moindre échelle, elle est active sur quelques mycoplasmes, sur les rickettsies et sur les spirochètes.

Le thiocyanate d'érythromycine est parfaitement toléré jusqu'à 4 000 mg/kg quand il est administré par voie orale à la souris ; chez cet animal, la DL50/24 h/kg se situe à 710 mg par voie intra-péritonéale, à 1 800 mg par voie sous-cutanée, à 650 mg par voie endo-veineuse et à 394 mg par voie intra-musculaire. Chez le cobaye, la DL50/24 h/kg est de 413 mg quand il est administré par voie intra-péritonéale.

Comme les autres macrolides, l'ERYTHROMYCINE se fixe sur la fraction 50 S des ribosomes empêchant la translocation, perturbant ainsi la synthèse des protéines de la cellule bactérienne. L'antibiotique est rapidement absorbé, qu'il soit administré par injection intra-mammaire, par voie digestive ou par voie parentérale. Le taux maximum est atteint dans les 6 heures suivant l'administration et il redevient pratiquement nul en 24 heures. Par voie parentérale ou mammaire, l'élimination se fait essentiellement par la bile alors que par voie orale une partie est éliminée sous forme active par les fèces.

Cet antibiotique est ainsi classiquement indiqué dans la plupart des infections à cocci à gram + et à cocci à gram (-), dans les infections à clostridies, à corynebactéries, à actinomycètes, à bacille du rouget, à staphylocoques, à Listeria, à Haemophilus, à Bordetella, à colibacilles, à Vibrio Coli, à leptospires, à rickettsiales, à mycoplasmes.

L'association des deux antibiotiques décrits ci-dessus est classiquement indiquée dans le traitement des affections microbiennes les plus fréquentes et les plus graves des mamelles bovines, les germes causaux pouvant être ainsi classés par ordre de fréquence décroissant :
STREPTOCOQUES, STAPHYLOCOQUES, ENTEROCOQUES, COLIBACILLES, CORYNE-BACTERIES, AEROBACTER AEROGENES, PSEUDOMONAS PYOCYANEUS, ainsi que SERRATIA MARCESSENS, CITROBACTER, PROTEUS MIRABILIS et KLEBSIELLA PNEUMONIAE.

Le lysozyme ou MURAMIDASE est une enzyme mucolytique cristalline, très largement répandue dans la nature. Elle est présente dans le blanc d'oeuf, le mucus nasal, la salive, le lait, les larmes, le sérum, les leucocytes, etc... C'est un polypeptide contenant une forte proportion d'acides aminés tels que l'arginine, l'acide aspartique, le tryptophane ; il est stable en solution acide et jusqu'à une température ne dépassant pas 55 °C.

Cette enzyme est active contre certaines bactéries gram + et contre quelques virus. Elle est parfaitement tolérée puisque des doses supérieures à 5 000 mg/kg/24 h, administrée par voie orale à la souris et au rat, n'ont provoqué aucun phénomène toxique.

Son poids moléculaire est de 14 500. Administrée par voie orale, elle est très peu absorbée dans le tractus digestif et très peu par le système lymphatique.

Cette enzyme aide l'organisme dans ses défenses vis-à-vis des infections bactériennes et virales et elle est recommandée dans le traitement des maladies bactériennes à gram + et à gram (-) notamment dans les affections respiratoires, cutanées, de la sphére ORL et des mammites.

Le médicament de l'invention, constitué d'une association de deux antibiotiques et d'une enzyme a pour autre caractéristique de traiter les mammites bovines et caprines par infusion, sous pression douce dans le sinus galactophore, d'une solution renfermant les deux antibiotiques associés au lysozyme.

Le médicament ainsi composé peut être présenté comme suit, la description ci-dessous étant donnée à titre d'exemple non limitatif, d'autres présentations pouvant bien évidemment être utilement employées : un sachet de matière plastique souple et résistante contient de la polymyxine en solution dans du soluté isotonique stérile et apyrogène à 9 p.1000 de chlorure de sodium : cette solution est ajustée à un pH convenable par addition d'une solution tampon phosphatée. Ce sachet, scellé à chaud, présente à l'une de ses extrémités un tube excréteur en matière plastique siliconée, qui peut avoir une longueur de un mètre et un diamètre de 5 mm, oblitéré par une capsule métallique. Dans ce liquide flotte un petit flacon qui peut être de verre ou de toute autre matière compatible avec le milieu, à large col muni d'une fermeture hermétique facilement décapsulable. Ce second récipient contient l'érythromycine en poudre et le lysozyme également présenté sous forme d'une poudre lyophilisée.

Le médicament de l'invention est présenté pour l'administration intra-mammaire sous forme de soluté. Le conditionnement particulier du médicament permet la mise en solution extemporanée de l'érythromycine et du lysoxyme, contenus sous forme ·de poudre dans un petit flacon, dans les 250 ml de solution contenant déjà la Polymyxine en solution tamponnée, le soluté final de la perfusion ayant ainsi un pH de 6,8, voisin de celui du lait normal. Cette présentation du médicament de l'invention permet une longue conservation des différents constituants et la libération de leur activité au moment de l'emploi. Il y a lieu également de préciser que le conditionnement du médicament de l'invention est un dispositif à usage unique, l'utilisation en une seule fois du contenant assurant au traitement une intégrité hygiénique et une facilité d'emploi incomparables.

Une autre variante de la présentation galénique du médicament de l'invention consiste à ce que le petit flacon flottant dans le sachet de matière plastique contienne les deux antibiotiques (erythromycine et polymyxine) et l'enzyme (lysozyme) de la préparation, le solvant contenu dans le sachet de matière plastique étant du soluté isotonique stérile et apyrogène de chlorure de sodium, tamponné à pH 6,8.

Dans ce cas, le flacon contenant les deux antibiotiques (erythromycine et polymyxine) et l'enzyme (lysozyme) de la préparation peut être indépendant de la poche plastique contenant le solvant tamponné à pH 6,8, la dissolution des antibiotiques et de l'enzyme dans ledit solvant est alors réalisée par un système approprié au moment de l'utilisation.

Une autre caractéristique de l'invention est représentée par la quantité du vecteur utilisé. En effet, alors que les produits classiquement administrés le sont sous forme de pommade, de crème ou de solution peu volumineuse, de l'ordre de 50 à 100 ml le médicament de l'invention est administré sous un volume minimum de 250 ml, cette administration étant rendue possible par perfusion. Cette quantité de liquide présente, par rapport aux traitements classiques, plusieurs avantages qui sont :

1 - une diffusibilité grande et rapide qui permet ainsi d'entraîner les principes actifs du médicament de l'invention en quelques heures jusqu'aux acini,

2 - une absorption rapide dans les circuits sanguins, ce qui provoque une résorption partielle des antibiotiques par voie interne, diminuant ainsi leur excrétion par la mamelle et la pollution du lait,

3 - une isotonicité à la préparation évitant l'éclatement des bactéries décapsulées par les principes actifs.

Des essais de toxicité aiguë ont été réalisés avec le médicament de l'invention sur deux espèces animales de laboratoire. Les résultats obtenus sont consignés ci-dessous. Ils montrent la faible toxicité du traitement en cause, comparée aux doses normales employées (250 ml/quartier et par jour), cette toxicité découlant de celle des principes actifs.

| Espèces Animales | Voies d'administration | $DL_0$/ml/kg | $DL_{100}$/ml/kg |
|---|---|---|---|
| Rat | Sous-cutanée | 50 | 150 |
| | Intra-péritonéale | 5 | 50 |
| | Intra-musculaire | 50 | 100 |
| Souris | Sous-cutanée | 50 | 150 |
| | Intra-péritonéale | 50 | 150 |
| | Intra-musculaire | 20 | 40 |

Il y a lieu de préciser que 1 ml de la préparation ayant fait l'objet des résultats précités contient 500 U.I. de polymyxine B sulfate, 0,3 mg de lysozyme chlorhydrate et 2 mg d'érythromycine thiocyanate exprimé en base.

Il est connu que, dans une préparation destinée au traitement de mammites et contenant plus de deux antibiotiques, il est très difficile de contrôler l'efficacité et l'élimination individuelle des principes actifs, malgré une efficacité certaine dans les premiers jours, les médicaments ne contenant que deux antibiotiques ont souvent permis une rechute clinique ou latente dès le quatrième jour après le traitement. C'est ainsi qu'à titre d'exemple, une mammite staphylococcique a présenté l'évolution suivante qui montre une rechute et la nécessité d'un nouveau traitement, la dose unique d'antibiotiques administrés étant de 500 mg d'ERYTHROMYCINE thiocyanate et de 125 000 U.I. de POLYMYXINE sulfate, sous un volume de 250 ml.

| JOUR DE L'ANALYSE | Nombre de germes | Nombre de cellules somatiques |
|---|---|---|
| Avant traitement | 15 000 | 12 540 000 |
| 1° traite après le traitement | 0 | 5 000 000 |
| 2° traite après le traitement | 6 000 | 2 500 000 |
| 3° traite après le traitement | 5 000 | 3 000 000 |
| 4° traite après le traitement | 6 000 | 3 500 000 |
| 5° traite après le traitement | 17 000 | incomptable |
| 6° traite après le traitement | 346 000 | incomptable |
| 7° traite après le traitement | 535 000 | incomptable |
| 8° traite après le traitement | 406 000 | incomptable |

Un autre exemple d'une mammite colibacillaire ancienne a présenté la même évolution, la dose d'antibiotiques administrée étant identique à l'exemple précédent.

Une vache hollandaise, âgée de 6 ans, est à terme mais n'a pas encore vêlé. En fin de période de tarissement, le quartier postérieur gauche a brusquement enflé. Aucun produit n'avait été administré dans les trayons de la vache à la dernière traite. Cette enflure du quartier a été traitée par l'éleveur à l'aide de plusieurs seringues antimammite sans résultat. Ce quartier présente une phlegmasie importante mais pas de douleur notable. Sa sécrétion se résume à quelques giclées de sérosité citrine. Il n'y a pas de perturbation de l'état général et la température rectale est à 38°5. Il est alors décidé de procéder à une perfusion mammaire avec le médicament de l'invention ; la perfusion est aisée et rapide et ne dure que 43 secondes. Le quartier désenfle en 24 heures : la vache a vêlé trois jours après le traitement et sa production totale dépasse 20 litres de lait par jour : il n'a été observé ni séquelles ni faiblesse du quartier traité. Sur le plan bactériologique, il a été noté la présence de Streptocoque avant traitement et de Citrobacter après traitement. Le résultat obtenu a été excellent, les mêmes avantages que précédemment étant également constatés, nonobstant un traitement antérieur par des procédés classiques, ce qui montre la supériorité du médicament de l'invention sur les traitements habituels.

Une vache frisonne, âgée de 8 ans, est atteinte, 3 jours après le vêlage du quartier postérieur gauche. Ce quartier avait été traité de manière prolongée à l'aide de l'association cloxacilline-colistine. L'affection a semblé guérir mais une rechute a été constatée au bout de cinq jours. Le quartier est enflé et induré et donne un lait décomposé contenant des caillots longs et fibrineux. La production de l'animal est diminuée d'au moins un tiers par rapport à celle des autres animaux. Il est alors décidé de traiter l'animal à l'aide du médicament de l'invention, cet animal ne recevant qu'un seul traitement, la perfusion étant aisée et ne durant que 43 secondes.

L'animal est revu le lendemain : le quartier a déjà désenflé et le lait à repris un aspect normal dès la traite du matin. Revu 15 jours plus tard, on constate que le quartier atteint n'a pas gardé de séquelle et que sa production est égale à celle des trois autres. La production totale de la vache est montée à 20 litres de lait par jour, ce qui est le niveau normal de l'animal en une semaine.

Le résultat obtenu, qui est excellent, montre l'efficacité du médicament de l'invention dans le traitement d'une mammite n'ayant pu être guérie par les traitements habituels.

Une vache normande, âgée de 8 ans, est atteinte depuis 15 jours de façon chronique du quartier avant droit qui n'est ni enflé, ni douloureux mais qui présente dans le parenchyme des nodules durs en noyau de pêche. Ce quartier qui paraît moins volumineux que les trois autres donne une sérosité aqueuse contenant de gros caillots ressemblant à du fromage blanc. La mammite a déjà été traitée à plusieurs reprises à l'aide de divers traitements mais la production totale de l'animal est passée de 18 à 3 l de lait par jour. Par ailleurs, l'état général n'est pas affecté par la mammite.

Il est alors décidé de procéder à une infusion mammaire avec le médicament de l'invention : la perfusion est aisée et rapide et ne dure que 46 secondes.

La vache, revue le lendemain, ne présente ni inflammation ni réaction locale du quartier traité dont la sécrétion s'est transformée en sérosité crémeuse mal liée. La production du quartier n'a pas varié.

L'inflammation du quartier disparaît complètement 4 jours après le traitement unique. Les nodules du parenchyme disparaissent également. Le lait reprend un aspect normal après 5 ou 6 traites et la production revient à la normale en une semaine.

Le résultat obtenu est donc excellent ; il est en faveur du médicament de l'invention qui se montre nettement supérieur aux traitements habituels.

Une vache hollandaise, âgée de 6 ans, est atteinte depuis une dizaine de jours du quartier postérieur gauche très enflé et douloureux et qui donne un lait crêmeux contenant des caillots. La production totale de l'animal n'est que de dix litres par jour. La mammite a déjà été traitée -sans résultat- à l'aide de différents médicaments dont des antibiotiques administrés par voie endoveineuse. Il n'y a pas de perturbation de l'état général et la température rectale est à 38°6.

Il est alors décidé de procéder à une perfusion mammaire avec le médicament de l'invention. Deux jours après, le quartier a considérablement désenflé et en quatre jours, le lait reprend un aspect normal, la production totale de l'animal passant alors à 15 litres de lait par jour. La vache est revue six semaines après. Le quartier postérieur gauche est tout-à-fait normal et on ne constate aucune induration résiduelle. La production totale atteint alors 25 litres par jour.

Le résultat obtenu a été excellent et la tolérance a été parfaite. Cette observation est également en faveur du médicament de l'invention qui s'est montré avoir une efficacité nettement supérieure à celle des traitements classiques.

Les résidus d'antibiotiques dans le lait revêtent une importance particulière, tant pour la santé des consommateurs que pour la technologie laitière et fromagère. Ils ont donc été recherchés dans le lait, soit après traitement par le médicament de l'invention, soit à titre de comparaison, après traitement avec une association de deux antibiotiques et d'une sulfamide. Les résultats des essais sont consignés, à titre d'exemple, ci-dessous :

VACHE A traitée avec le médicament de l'invention

| Dosage des antibiotiques / Nombre d'heures après traitement | ERYTHROMYCINE | | POLYMYXINE | |
|---|---|---|---|---|
| | Quartiers traités | Quartiers non traités | Quartiers traités | Quartiers non traités |
| 6 heures | 21,36 UI/ml | O | 305 UI/ml | O |
| 12 heures | 1,26 UI/ml | O - | O | O |
| 24 heures | O | O | O | O |

VACHE B traitée avec une pommade contenant FRAMYCETINE + CHLORAMPHENIOL + SULFAPYRIDAZINE (dosage par la méthode de LAGODSKY de diffusion en gélose avec SARCINA LUTEA)

| Nombre d'heures après traitement | DOSAGES | |
|---|---|---|
| | Quartiers traités | Quartiers non traités |
| 6 heures | ⟩ 40 µg/ml | ⟨0,25 et 0,12 µg/ml |
| 12 heures | 30 µg/ml | ⟨0,06 µg/ml |
| 24 heures | 20 µg/ml | ⟨0,03 µg/ml |
| 36 heures | 9 µg/ml | 0 |
| 48 heures | 0,5 µg/ml | 0 |
| 60 heures | ⟨0,5 et ⟩0,25 µg/ml | 0 |
| 72 heures | ⟨0,25 et ⟩ 0,12 µg/ml | 0 |
| 84 heures | ⟨0,03 µg/ml | 0 |

L'examen de ces résultats est en faveur du médicament de l'invention. En effet, alors que la pollution antibiotique n'affecte que la première traite après traitement, parfois la deuxième mais jamais la troisième, on constate qu'avec les traitements habituels, le lait des quartiers traités contient des résidus d'antibiotiques jusqu'à la septième traite suivant la dernière administration.

On donnera, ci-après, à titre d'exemple non limitatif, une formulation pharmaceutique du médicament de l'invention pour 100 ml de soluté final :

1 - flacon contenant :

    - érythromycine thiocyanate

    (exprimés en base)..................... 200 mg

14

   - lysozyme chlorhydrate............  30 mg

2 - <u>Solvant</u> :

   - polymyxine B sulfate........... 50 000 U.I.

   - sérum physiologique tamponné

     à pH 6,8..........q.s.p...  100 ml

   (au tampon phosphate 0,05 M)

Un autre exemple de formulation peut être le suivant :

   - 1 - flacon contenant :

    -polymyxine E sulfate.......250 000 U.I.

    -érythromycine thiocyanate

     (exprimée en base)....  500 mg

    -lysozyme chlorhydrate......  75 mg

   - 2 - solvant :

    -soluté isotonique de chlorure

    de sodium tamponné à pH 6,8

      ...q.s.p....  250 ml

Une autre formulation du médicament de l'invention contient -en lieu et place de 50 000 U.I de polymyxine B sulfate- 75 000 U.I. de polymyxine E sulfate.

Chaque dose unitaire de 250 ml peut contenir avantageusement entre 400 et 600 mg d'érythromycine exprimée en base, de 75 à 100 mg de lysozyme exprimée en base et de 50 000 à 250 000 U.I. de polymyxine exprimée en base.

Les études toxicologiques, pharmacologiques et cliniques qui viennent d'être rapportées ont mis en évidence la bonne tolérance du médicament de l'invention ainsi que ses activités anti-inflammatoires, bactéricides et bactériostatiques.

Le médicament de l'invention peut ainsi être administré à l'espèce bovine avec profit dans le traitement des mammites cliniques aiguës, subaiguës ou récidivantes d'étiologie infectieuse à germes gram + ou gram (-).

0097580

15

R E V E N D I C A T I O N S

1 - Nouveau médicament à usage vétérinaire possédant une activité antimicrobienne, contenant à titre de principe actif deux antibiotiques associés à une enzyme et caractérisé en ce que les antibiotiques sont la polymyxine et l'erythromycine ou leurs sels pharmaceutiquement acceptables, et que l'enzyme est le lysozyme ou ses sels pharmaceutiquement acceptables.

2 - Médicament selon la revendication 1 caractérisé en ce que les principes actifs sont administrés en solution ayant un pH final voisin de celui du lait normal.

3 - Médicament selon la revendication 2, caractérisé en ce que la quantité de vecteur utilisée pour chaque dose unitaire a un volume minimum de 250 ml.

4 - Médicament selon une des revendications 1 à 3, caractérisé en ce que chaque dose unitaire de 250 ml contient avantageusement entre 400 et 600 mg d'érythromycine exprimée en base, de 75 à 100 mg de lysozyme exprimée en base et de 50 000 à 250 000 U.I. de polymyxine exprimée en base.

5 - Médicament selon une des revendications 1 à 4, caractérisé en ce qu'il doit être administré par infusion, sous pression douce, dans le sinus galactophore des glandes mammaires.

6 - Médicament selon une des revendications 1 à 5, caractérisé en ce qu'il doit être administré à l'espèce bovine et à l'espèce caprine dans le traitement des mammites aiguës, subaiguës, récidivantes, d'étiologie infectieuse.

Office européen
des brevets

RAPPORT DE RECHERCHE EUROPEENNE

0097580
Numéro de la demande

EP 83 40 1232

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | UNLISTED DRUGS, vol. 23, no. 3, mars 1971, page 42, point c, Chatham, New Jersey, USA * Page 42c "Rubibacter" * | 1-6 | A 61 K 37/54 // (A 61 K 37/54 A 61 K 37/02 A 61 K 31/71 ) |
| A | UNLISTED DRUGS, vol. 23, no. 9, septembre 1971, page 129, point q, page 130, point h, Chatham, New Jersey, USA * Page 129q "Dibiosol", page 130h "Ecolicin" * | 1-6 | |
| A | CHEMICAL ABSTRACTS, vol. 72, no. 7, 16 février 1970, page 81, no. 29180f, Columbus, Ohio, USA Y. NAGAI et al.: "Combined antimicrobial effect of lysozyme with some antibiotics" & NIPPON KAGAKU RYOHOGAKUKAI ZASSHI 1969, 17(8), 1593-7 * Abrégé * | 1-6 | |
| A | DICTIONNAIRE VIDAL, 1971, pages 1170,1049, O.V.P, Paris, FR. * Page 1170 "Polyfra Pommade P.O.S."; page 1049 "Ophtabactil" * | 1-6 | |

DOMAINES TECHNIQUES RECHERCHES (Int. Cl. ³)

A 61 K

--- -/-

Le présent rapport de recherche a été établi pour toutes les revendications

| Lieu de la recherche LA HAYE | Date d'achèvement de la recherche 16-09-1983 | Examinateur BRINKMANN C. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82

## Office européen
## des brevets

# RAPPORT DE RECHERCHE EUROPEENNE

| | DOCUMENTS CONSIDERES COMME PERTINENTS | | Page 2 |
|---|---|---|---|
| Catégorie | Citation du document avec indication, en cas de besoin, des parties pertinentes | Revendication concernée | CLASSEMENT DE LA DEMANDE (Int. Cl. ³) |
| A | CHEMICAL ABSTRACTS, vol. 90, no. 7, 12 février 1979, page 109, no. 49022f, Columbus, Ohio, USA O.V. BUKHARIN et al.: "Experimental study of the antimicrobial effect of lysozyme in combination with antibiotics" & ANTIBIOTIKI (MOSCOW) 1978, 23(11), 997-1002 * Abrégé * | 1-6 | |
| | --- | | |
| A | FR-M- 4 842 (SCIENCE UNION ET CIE.) | 1-6 | |
| | --- | | |
| A | FR-M- 4 559 (ETABLISSEMENTS WANDER) | 1-6 | |
| | --- | | |
| A | GB-A- 792 544 (AMERICAN HOME PRODUCTS CORPORATION) | 1-6 | |
| | ----- | | DOMAINES TECHNIQUES RECHERCHES (Int. Cl. ³) |

Le présent rapport de recherche a été établi pour toutes les revendications

| Lieu de la recherche | Date d'achèvement de la recherche | Examinateur |
|---|---|---|
| LA HAYE | 16-09-1983 | BRINKMANN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82